# EUROPÄISCHE PATENTSCHRIFT

(11) **EP 2 007 456 B1**
(45) Veröffentlichungstag und Bekanntmachung des Hinweises auf die Patenterteilung: **17.10.2018**
(21) Anmeldenummer: 07727793.7
(22) Anmeldetag: 04.04.2007
(51) Int. Cl.: A61M 15/00

(54) **MUNDSTÜCK FÜR EINEN INHALATOR**
MOUTHPIECE FOR AN INHALER
EMBOUT BUCCAL POUR INHALATEUR

(30) Priorität: 11.04.2006 DE 102006016901
(43) Veröffentlichungstag der Anmeldung: 31.12.2008
(60) Teilanmeldung: 13176790.7
(73) Patentinhaber: Boehringer Ingelheim Pharma GmbH & Co. KG, 55216 Ingelheim am Rhein (DE)
(72) Erfinder: WACHTEL, Herbert, 55218 Ingelheim Am Rhein (DE)
(74) Vertreter: Simon, Elke Anna Maria
(86) Internationale Anmeldenummer: PCT/EP2007/053324
(87) Internationale Veröffentlichungsnummer: WO 2007/118800

(56) Entgegenhaltungen:
- WO-A-94/06498
- WO-A-02/056948
- CA-A1- 2 207 219
- DE-A1- 10 126 924
- US-A1- 2002 005 195

## Beschreibung

Die Erfindung bezieht sich auf ein Mundstück für einen Inhalator zur Verabreichung von einem Arzneimittel in Form inhalationsfähiger Substanzen, Substanzformulierungen oder -mischungen, das einen Inhalationskanal zur Kopplung mit einer Kammer zur Aufnahme des Arzneimittel aufweist, und einen Inhalator dazu.

Die EP 0 911 047 A1 offenbart einen Inhalator für die Inhalation pulverförmiger Arzneimittel aus Kapseln, der ein Unterteil mit zwei Fenstern und eine Platte, in der sich Kapselhalterungen sowie Lufteinlassöffnungen befinden, umfasst. Im Weiteren ist eine Inhalationskammer mit der Platte verbunden, an der ein mit zwei geschliffenen Nadeln versehener, gegen eine Feder beweglicher Kopf vorgesehen ist. Ein Mundrohr ist mit einem Oberteil des Inhalators und ein Deckel klappbar mit dem Unterteil, der Platte sowie dem Oberteil verbunden.

Des Weiteren beschreiben die DE 39 27 170 A1 und DE10126924 einen Inhalator für die Inhalation pulverförmiger, insbesondere mikronisierter Arzneimittel aus Kapseln, in dessen Gehäuse für die Aufnahme der Kapseln eine rohrförmige Kammer mit einem bodenseitigen Lufteinlass und einem in ein Mundstück übergehenden Luftauslass an dem gegenüberliegenden Kammerende und eine Schneideinrichtung mit zwei in dem Kammerinnenraum bewegbaren Schneiden zum Öffnen der Kapseln in der Nähe von deren oberen und unteren Ende angeordnet sind. Im oberen Bereich der Kammer, in der sie in den Inhalationskanal übergeht, ist eine Siebplatte angeordnet, die Teil eines trichterförmigen Verbindungsstückes ist, das auf den Anfang des Inhalationskanals derart aufsteckbar ist, dass der Trichterrand mit der Siebplatte in eine Einsatzplatte eingreift, die den Boden des Mundstückes bildet. Alternativ ist die Siebplatte im Klemmsitz zwischen dem Trichterrand des Verbindungsstückes und einem Anschlag der Einsatzplatte austauschbar befestigt. Dieser Inhalator ist insofern zu inhalierenden Aerosols im Inhalationskanal mit einer damit einhergehenden Deposition des Arzneimittels nicht auszuschließen ist.

Es ist Aufgabe der Erfindung, einen Inhalator der eingangs genannten Art zu schaffen, mit denen jeweils eine verbesserte Ausbringung des Arzneimittels gewährleistet ist.

Erfindungsgemäß wird die Aufgabe dadurch gelöst, dass in dem Inhalationskanal mindestens ein Bauteil mit einem aerodynamischen Querschnitt vorgesehen ist.

Aufgrund dieser Maßnahme ist die unkontrollierte Verwirbelung des zu inhalierenden Aerosols im Inhalationskanal verringert und unhygienisch erscheinende Rückstände des Arzneimittels im Bereich des Mundstückes werden verringert. Dem Fachmann sind aus der Strömungstechnik hinreichend Profile mit einem aerodynamischen Querschnitt bekannt, mit denen sich eine verhältnismäßig geringe Verwirbelung eines Aerosols beim Inhalieren realisieren lässt.

Inhalatoren sind unter den Markennamen HandiHaler®, Spinhaler®, Rotahaler®, Aerolizer®, Flowcaps®, Turbospin®, AIR DPI®, Orbital®, Directhaler® bekannt und/oder in DE 33 45 722, EP 0 591 136, DE 43 18 455, WO 91/02558, FR-A-2 146 202, US-A-4 069 819, EP 666085, EP 869079, US 3,991,761, WO 99/45987, WO 200051672, Bell, J. Pharm. Sci. 60, 1559 (1971); Cox, Brit. Med. J. 2, 634 (1969), beschrieben. Als Pulverinhalatoren sind Einfach- oder Mehrfachdosis-Pulverinhalatoren, insbesondere der Spinhaler®, Rotahaler®, Aerolizer®, Inhalator®, HandiHaler®, Diskhaler®, Diskus®, Accuhaler®, Aerohaler®, Eclipse®, Turbohaler®, Turbuhaler®, Easyhaler®, Novolizer®, Clickhaler®, Pulvinal®, Novolizer®, SkyeHaler®, Xcelovair®, Pulvina®, Taifun®, MAGhaler®, Twisthaler® und der Jethaler® bekannt.

Die WO 94/06498 A betrifft beispielsweise ein medizinisches Inhalationsgerät mit Gehäuse und Mundstück. Dieses Gerät umfasst ein Antriebselement, das im Innern des Geräts auf einer Achse koaxial zur Längsachse des Gehäuses drehbar gelagert ist und nahe dem Lufteinlass des Geräts eine Aufnahme zur Kapselhalterung aufweist. Rotorblätter am Antriebselement ragen derart in die Luftströmungsdurchlässe hinein, dass das drehbar gelagerte Antriebselement bei einer durch Einatmung des Patienten erzeugten Luftströmung in Rotation und Vibration versetzt wird. Die fest am Antriebselement angebrachte Kapsel wird dabei mitbewegt und gibt durch Löcher Medikament ab, das vom Luftstrom mitgerissen und vom Patienten inhaliert wird. Die Löcher werden zuvor mit im Gerät vorhandenen Anstechnadeln in die eingesetzte Kapsel gestochen.

Die WO 02/056948 A zeigt beispielsweise einen Pulver-Inhalator, in dem ein durch Druckgas im Gerät erzeugter Gasstrom loses Pulver durch Auftreffen oder Überströmen mitreißt. Die so erzeugte Gas-Pulver-Dispersion trifft auf einen Prallkörper auf, bevor sie zum Mundstück gelangt. Durch Auftreffen auf den Prallkörper werden Partikel weiter deagglomeriert, und die Geschwindigkeit des Gasstroms wird dort abgebremst.

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Anti-histaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar; kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmern oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-{4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethy]-2-(2,4,6-trimethy]phenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 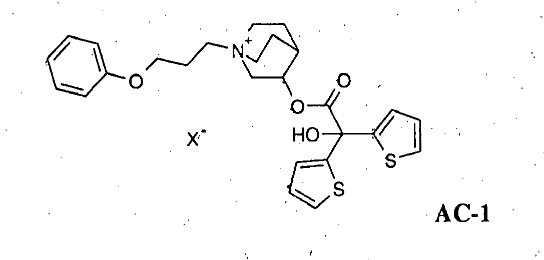 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 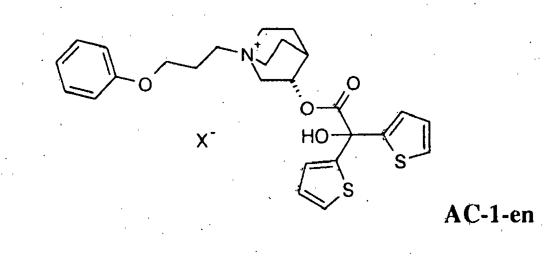 enthalten, worin X⁻ die vorstehend genannten Bedeutungen aufweisen kann. Weiterhin bevorzugte Anticholinergika sind ausgewählt aus den Salzen der Formel **AC-2** 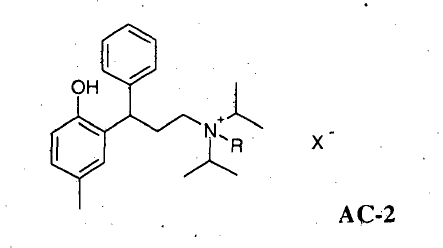 worin R entweder Methyl oder Ethyl bedeuten und worin X⁻ die vorstehend genannte Bedeutungen aufweisen kann. In einer alternativen Ausführungsform kann die Verbindung der Formel **AC-2** auch in Form der freien Base **AC-2-base** vorliegen.

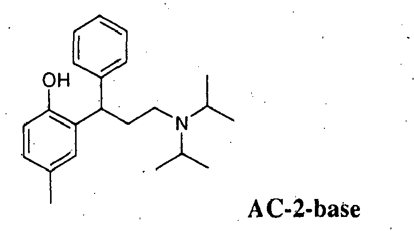

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluören-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Metbobromid
Die vorstehend genannten Verbindungen sind im Rahmen der vorliegenden Erfindung auch als Salze einsetzbar, in denen statt des Methöbromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-35-yl)ester,
- 6α,9α-difluoro-11β-hydroxy-16α-methyl-3-oxo-17α-(2,2,3,3-tertamethylcyclo-propylcarbonyl)oxy-androsta-1,4-diene-17β-carbonsäure cyanomethyl ester gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, A-riflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure] 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]-amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-Chinazolin
- 4-[(3-Chlor-4-fluor-phenyl)amino]-6-{[4-((R)-6-methy]-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[-N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethytamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin.
- 4-[(3-Chlor-4-fluorphenyl)amino]-7-[3-(morpholin-4-yl)-propyloxyl-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{(4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethy]-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-(2-[4-(2-oxo-morpholin-4-yl)-piperidin-1-yl]-ethoxy}-7-methoxy-chinazolin
- 4-[(3-Chlor-4-fluor-phenyl)amino]-6[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yl-oxy}-7-methoxy-chinazolin
- 4-[(3-Chlor-4-fluor-phenyl)amino]-6-{1-[(methoxymethyl)carbonyl]-piperidin-4-yl-oxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yl-oxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino)-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-y]oxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethiny)-phenyl)amino]-6-[1-(2-methoxy-acetyl)-pipendin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Ch]or-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergölin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkomalkaloide: Dihydroergotamin, Ergotamin.

Für die Inhalation kommen Arzneimittel mit den o.g. Wirkstoffen in Betracht, sowie deren Salze, Ester sowie die Kombination dieser Wirkstoffe, Salze und Ester.

Bevorzugt ist das Bauteil einstückig mit dem Inhalationskanal verbunden und erstreckt sich radial in den Inhalationskanal. Damit lassen sich Kosten bei der Montage des Mundstückes bzw. Inhalators einsparen.

Nach einer Weiterbildung ist das Bauteil als Stromlinienkörper, insbesondere NACA-Profil, ausgebildet. Bei einem NACA-Profil handelt es sich um ein symmetrisches Profil einer Versuchsreihe, die das National Advisory Commitee for Aerodynamic (NACA) durchgeführt hat. NACA-Profile zeichnen sich durch eine abgerundete Nase und ein spitz auslaufendes hinteres Teil aus. Unterschiedliche NACA-Profile sind bespielsweise in Dubs, Fritz, Aerodynamik der reinen Unterschallströmung, 4. neubearb. Auflage, Birkhäuser Verlag Basel, ISBN 3-7643-1073-1, auf den Seiten 210 bis 213, 218, 219, 222 und 223 beschrieben.

Vorzugsweise wird ein NACA-Profil 0030 oder ein ähnliches Profil verwendet, wobei sich zeigte, dass die abgegebene Dosis des Arzneimittels durch die Verwendung eines solchen Profils gegenüber einem konventionellen Sieb vergrößert ist. Zweckmäßigerweise weist die abgerundete Nase des Stromlinienkörpers in Richtung des Mundstücks und das spitze Ende zur Kammer.

Um zu verhindern, dass relativ große Partikel duch den Inhalationskanal gelangen und darüber hinaus einen Anschlag für eine das Arzneimittel aufnahmende Kapsel bereitzustellen, sind mehrere Stromlinienkörper sternförmig zueinander ausgerichtet. Durch diese Anordnung ist auch eine verhältnismäßig hohe Stabilität gewährleistet, die zur Entleerung der bei der Inhalation vibrierenden Kapsel beiträgt. Im Weiteren lässt sich mit der sternförmigen Ausrichtung der NACA-Profile ein zyklonartiger Wirbel beim Inhalieren erzeugen, der zur Modifikation der Teilchengrößenverteilunng des Arzneimittels eingesetzt werden kann, da ein starker Wirbel zu einer bevorzugten Deposition schwerer und großer Partikel im Inhalationskanal führt, während leichte Wirkstoffteilchen zum Patienten bzw. Benutzer des Inhalators gelangen. Bevorzugt ist hierbei der Inhalationskanal im Querschnitt rund ausgebildet. Zweckmäßigerweise sind die Stromlinienkörper an dem der Mundseite gegenüberliegenden Seite des Inhalationskanals angeordnet.

In Ausgestaltung besteht das Mundstück mitsamt dem Inhalationskanal und den NACA-Profilen aus einem Kunststoff. Vorzugsweise handelt es sich bei den Kunststoffen um Polymerisate, thermoplastische Polykondensate, Polyaddukte, abgewandelte Naturstoffe oder Kautschuke bzw. um Gemische dieser Kunststoffe. Besonders bevorzugt sind hier Polyolefine, Vinylchlorid-Pölymerisate, Styrol-Polymerisäte, Polyacetale, Polyamide, thermoplastische Polyester und Polyarylether bzw. Gemische dieser Kunststoffe. Beispiele für diese Kunststoffe sind Polyethylen, Polyvinylchlorid, Polyoxymethylen, Polyacetal, Acrylnitril/Butadien/Styrol(ABS), Acrylnitril/Styrol/Acryl-ester(ASA), Polyamide, Polycarbonat, Poly (ethylenterephthalat), Poly(butylenterephthalat) oder Poly(phenylenether). Derartige Kunststoffe können beispielsweise von der Firma Ensinger in Deutschland, Nufringen, bezogen werden.

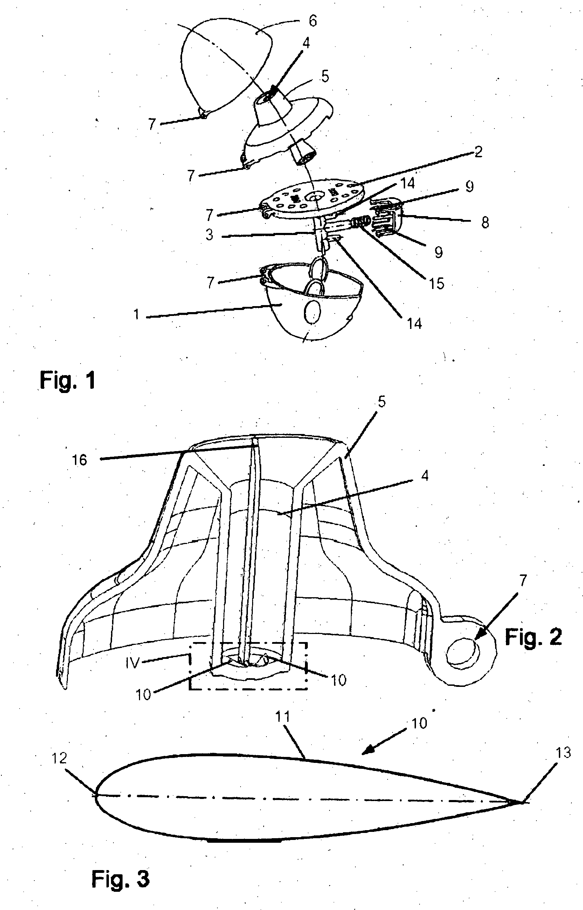

Vorzugsweise ist das Mundstück mitsamt dem Inhalationskanal und den darin angeordneten NACA-Profilen im Spritzgussverfahren herstellbar. Die NACA-Profile dienen hierbei als Verteiler für die Kunststoffschmelze, wobei ein Anspritzpunkt beispielsweise im Zentrum der sternförmig zueinander angeordneten Profile ausgebildet sein kann. Außer einer sternförmigen Anordnung ist in einer weiteren Ausführungsform auch eine bügelartige Verbindung der Stege im Mundstückinnenkanal möglich (Fig. 2).

Das zuvor beschriebene Mundstück findet bevorzugt in einem Inhalator für die Inhalation eines pulverförmigen Arzneimittels aus einer Kapsel Verwendung, wobei der Inhalator ein Unterteil umfasst, an dem eine mit dem Unterteil verrastbare Platte zum Verschließen des Unterteils angelenkt ist Die Platte weist eine in dem Unterteil versenkbare Kapselhalterung zur Aufnahme der Kapsel auf und ist mit dem Mundstück verrastbar. Ein Deckel, der das Mundstück in einer Verschlussposition abdeckt, ist mittels eines Verschlusselementes verrästet. Das Unterteil, die Platte, das Mundstück und der Deckel sind durch ein einziges Gelenk verschwenkbar miteinander verbunden. Darüber hinaus ist ein Betätigungsorgan vorgesehen, das aus einer Ruhestellung in eine Bewegung versetzbar ist und dabei mit mindestens einer in die Kapselhalterung einstoßbaren Nadel zusammenwirkt. Bevorzugt sind zwei zueinander beabstandete Nadeln vorgesehen.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispieles unter Bezugnahme auf die zugehörigen Zeichnungen näher erläutert. Es zeigt:
- Fig. 1: eine perspektivische Teil-Schnittdarstellung eines Inhalators mit einem erfindungsgemäßen Mundstück,
- Fig.2: eine perspektivische Darstellung eines Schnittes durch das Mundstück nach Fig. 1,
- Fig.3: einen Querschnitt durch ein aerodynamisches Bauteil des Mundstücks gemäß der Darstellung nach Fig. 2 und

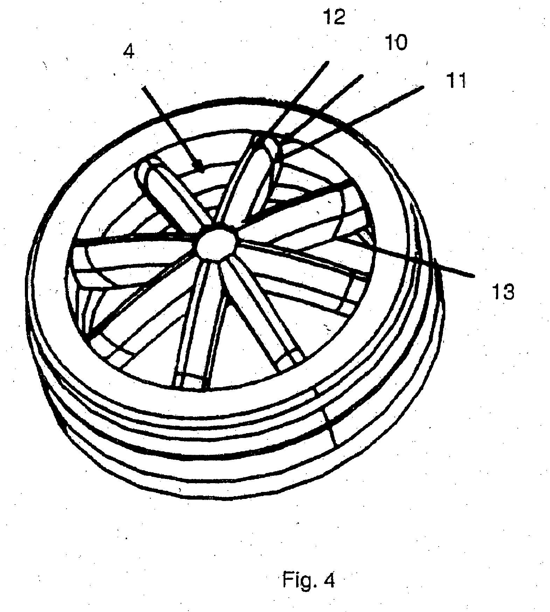

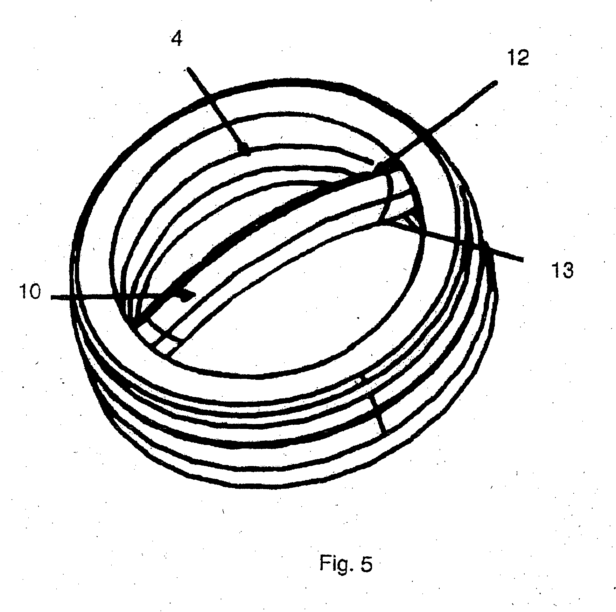

- Fig.4: eine vergrößerte perspektivische Darstellung der Einzelheit IV nach Fig. 2.

Der Inhalator weist ein Unterteil 1 auf, an dem eine mit dem Unterteil 1 verrastbare Platte 2 zum Verschließen des Unterteils 1 angelenkt ist. Die Platte 2 ist mit einer in dem Unterteil 1 versenkbaren Kapselhalterung 3 zur Aufnahme einer mit einem pulverförmigen Arzneimittel gefüllten Kapsel in einer Kammer versehen und mit einem einen Inhalationskanal 4 aufweisenden Mundstück 5 verrastbar. Ein Deckel 6, der das Mundstück 5 in einer Verschlussposition abdeckt, ist mittels eines Verschlusselementes verrastet. Das Unterteil 1, die Platte 2, das Mundstück 5 und der Deckel 6 sind durch ein gemeinsames Gelenk 7 verschwenkbar miteinander verbunden. Darüber hinaus ist ein Betätigungsorgan 8 vorgesehen, das mit zwei in die Kapselhalterung 3 einstoßbaren Nadeln 9 zum Einstechen der Kapsel zusammenwirkt. Die zueinander beabstandeten Nadeln 9 sind in Führungsstutzen 14 der Kapselhalterung 3 verschiebbar gelagert. Um eine Rückstellbewegung der Nadeln 9 zur Freigabe der Löcher in der Kapsel nach dem Loslassen des Betätigungsorgans 8 zu bewirken, ist zwischen der Kapselhalterung 3 und dem Betätigungsorgan 8 eine Druckfeder 15 angeordnet.

Das Mundstück 5 besteht mit dem darin ausgebildeten im Querschnitt runden Inhalationkanal 4 aus einem Kunststoff und ist im Spritzgussverfahren gefertigt. An dem der Mundseite gegenüberliegenden Seite des Inhalationskanals 4 sind mehrere sternförmig zueinander ausgerichtete Bauteile 10 mit einem aerodynamischen Querschnitt angeordnet. Die Bauteile 10 sind als NACA-Profile 11 ausgeführt und weisen jeweils eine abgerundete Nase 12 sowie ein spitzes Ende 13 auf, wobei das Ende auch abgerundet sein kann, um die Herstellung zu vereinfachen. Das Mundstück besitzt außerdem einen oder mehrere Stege 16, die das missbräuchliche Einführen von nicht geeigneten Kapseln verhindern, weiterhin das Strömungsverhalten des Inhalators und die Austrittsgeschwindigkeit des Arzneimittels günstig beeinflussen.

## Patentansprüche

1. Inhalator zur Verabreichung von einem pulverförmigen Arzneimittel in Form inhalationsfähiger Substanzen, Substanzformulierungen oder -mischungen aus einer Kapsel, wobei der Inhalator ein Mundstück (5) aufweist, das einen Inhalationskanal (4) zur Kopplung mit einer Kammer zur Aufnahme der Kapsel mit dem Arzneimittel aufweist, **dadurch gekennzeichnet, dass** in dem Inhalationskanal (4) mindestens ein Bauteil (10) mit einem aerodynamischen Querschnitt vorgesehen ist, wobei dieses Bauteil (10) als Stromlinienkörper ausgebildet ist, und dass das Bauteil (10) einstückig mit dem Inhalationskanal (4) verbunden ist und sich radial in den Inhalationskanal (4) erstreckt.

2. Inhalator nach Anspruch 1, **dadurch gekennzeichnet, dass** die abgerundete Nase (12) des Stromlinienkörpers in Richtung des Mundstücks (5) und ein spitzes Ende (13) zur Kammer weist.

3. Inhalator einem der vorangegangenen Ansprüche, **dadurch gekennzeichnet, dass** mehrere Stromlinienkörper sternförmig zueinander ausgerichtet sind.

4. Inhalator nach einem der vorangegangenen Ansprüche, **dadurch gekennzeichnet, dass** die Stromlinienkörper an dem der Mundseite gegenüberliegenden Seite des Inhalationskanals (4) angeordnet sind.

5. Inhalator nach einem der Ansprüche 1 bis 4, **dadurch gekennzeichnet, dass** es aus einem Kunststoff besteht.

6. Inhalator nach einem der Ansprüche 1 bis 5, **dadurch gekennzeichnet, dass** es im Spritzgussverfahren herstellbar ist.

7. Inhalator nach den Ansprüchen 4, 5 und 6, **dadurch gekennzeichnet, dass** im Mundstück ein im Querschnitt runder Inhalationskanal (4) ausgebildet ist und das Mundstück mindestens einen Steg (16) aufweist und sich dieser Steg (16) an der Innenwand des Inhalationskanals (4) befindet.

8. Inhalator nach einem der Ansprüche 1 bis 7 für die Inhalation eines pulverförmigen Arzneimittels aus einer Kapsel mit
- einem Unterteil (1)
- einer mit dem Unterteil (1) verrastbaren Platte (2) zum Verschließen des Unterteils (1), mit der das Mundstück (5) verrastbar ist, und eine in dem Unterteil (1) versenkbare Kapselhalterung (3) zur Aufnahme der Kapsel,
- einem Deckel (6), der das Mundstück (5) in einer Verschlussposition abdeckt und mittels eines Verschlusselementes verrastet, wobei das Unterteil (1), die Platte (2), das Mundstück (5) und der Deckel (6) durch ein einziges Gelenk (7) verschwenkbar miteinander verbunden sind, und
- einem Betätigungsorgan (8), das aus einer Ruhestellung in eine Bewegung versetzbar ist und dabei mit mindestens einer in die Kapselhalterung (3) einstoßbaren Nadel (9) zusammenwirkt.

9. Inhalator nach einem der Ansprüche 1 bis 8 zur Applikation eines pulverförmigen Arzneimittels mit einem Wirkstoff aus der Gruppe der Anticholinergika, Betamimetika, Steroide, Phosphodiesterase IV-inhibitoren, LTD4-Antagonisten, EGFR-Kinase-Hemmer, Antiallergika, Derivate von Mutterkornalkaloiden, Triptane, CGRP-Antagonisten, Phosphodiesterase-V-Inhibitoren, sowie Kombinationen aus solchen Wirkstoffen.

## Claims

1. Inhaler for administering a powdered medicament in the form of inhalable substances, substance formulations or substance mixtures from a capsule, the inhaler comprising a mouthpiece (5) which comprises an inhalation channel (4) for coupling to a chamber for receiving the capsule containing the medicament, **characterised in that** at least one component (10) having an aerodynamic cross section is provided in the inhalation channel (4), said component (10) being designed as a streamlined body, and **in that** the component (10) is integrally connected to the inhalation channel (4) and extends radially into the inhalation channel (4).

2. Inhaler according to claim 1, **characterised in that** the rounded nose (12) of the streamlined body points towards the mouthpiece (5) and a pointed end (13) points towards the chamber.

3. Inhaler according to either of the preceding claims, **characterised in that** a plurality of streamlined bodies are orientated with respect to one another so as to form a star.

4. Inhaler according to any one of the preceding claims, **characterised in that** the streamlined bodies are arranged at the end of the inhalation channel (4) that is opposite the mouth end.

5. Inhaler according to any one of claims 1 to 4, **characterised in that** it consists of a plastics material.

6. Inhaler according to any one of claims 1 to 5, **characterised in that** it can be produced by an injection moulding process.

7. Inhaler according to claims 4, 5 and 6, **characterised in that** an inhalation channel (4) that is round in cross section is formed in the mouthpiece, the mouthpiece comprises at least one connecting piece (16), and said connecting piece (16) is located on the inner wall of the inhalation channel (4).

8. Inhaler according to any one of claims 1 to 7 for the inhalation of a powdered medicament from a capsule, comprising:
- a lower portion (1)
- a panel (2) that can be locked to the lower portion (1) for closing off the lower portion (1), to which panel the mouthpiece (5) can be locked, and a capsule holder (3) that can be embedded in the lower portion (1) in order to receive the capsule,
- a cover (6) that covers the mouthpiece (5) in a closed position and locks said mouthpiece by means of a closing element, the lower part (1), the panel (2), the mouthpiece (5) and the cover (6) being pivotably interconnected by means of a single hinged joint (7), and
- an actuator (8) that can be shifted into movement from an inactive position and in the process interacts with at least one needle (9) that can be pushed into the capsule holder (3).

9. Inhaler according to any one of claims 1 to 8 for applying a powdered medicament having an active ingredient from the group consisting of anticholinergics, betamimetics, steroids, phosphodiesterase-4 inhibitors, LTD4 antagonists, EGFR kinase inhibitors, anti-allergic agents, ergot alkaloid derivatives, triptans, CGRP antagonists, phosphodiesterase-5 inhibitors, and combinations of active ingredients of this kind.

## Revendications

1. Inhalateur pour l'administration d'un médicament sous forme pulvérulente sous la forme de substances pouvant être inhalées, de formulations ou de mélanges de substances d'une capsule, dans lequel l'inhalateur présente un embout buccal (5) qui présente un canal d'inhalation (4) pour le couplage avec une chambre pour la réception de la capsule avec le médicament, **caractérisé en ce que** dans le canal d'inhalation (4) au moins un élément (10) comportant une section aérodynamique est prévu, dans lequel ledit élément (10) est conçu comme un profil aérodynamique, et **en ce que** l'élément (10) est relié d'un seul tenant avec le canal d'inhalation (4) et s'étend radialement dans le canal d'inhalation (4).

2. Inhalateur selon la revendication 1, **caractérisé en ce que** le nez arrondi (12) du profil aérodynamique est tourné en direction de l'embout buccal (5) et une extrémité pointue (13) est tournée vers la chambre.

3. Inhalateur selon l'une des revendications précédentes, **caractérisé en ce que** plusieurs profils aérodynamiques sont orientés les uns par rapport aux autres en forme d'étoile.

4. Inhalateur selon l'une des revendications précédentes, **caractérisé en ce que** le profil aérodynamique est disposé sur le côté opposé au côté buccal du canal d'inhalation (4).

5. Inhalateur selon l'une des revendications 1 à 4, **caractérisé en ce qu'**il est constitué d'une matière plastique.

6. Inhalateur selon l'une des revendications 1 à 5, **caractérisé en ce qu'**il peut être fabriqué dans un procédé de coulage par injection.

7. Inhalateur selon les revendications 4, 5 et 6, **caractérisé en ce qu'**un canal d'inhalation (4) de section arrondie est formé dans l'embout buccal et l'embout buccal présente au moins une traverse (16) et cette traverse (16) se trouve sur la paroi intérieure du canal d'inhalation (4).

8. Inhalateur selon l'une des revendications 1 à 7 pour l'inhalation d'un médicament pulvérulent d'une capsule comportant
- une partie inférieure (1)
- une plaque (2) pouvant être encliquetée avec la partie inférieure (1) pour la fermeture de la partie inférieure (1), avec laquelle l'embout buccal (5) peut être encliqueté, et un support de capsule (3) escamotable dans la partie inférieure (1) pour la réception de la capsule,
- un couvercle (6) qui recouvre l'embout buccal (5) dans une position de fermeture et s'encliquette au moyen d'un élément de fermeture, dans lequel la partie inférieure (1), la plaque (2), l'embout buccal (5) et le couvercle (6) sont reliés les uns aux autres par une articulation (7) unique pouvant basculer, et
- un organe d'actionnement (8) qui peut être déplacé d'une position de repos à un mouvement et coopère ainsi avec au moins une aiguille (9) pouvant être introduite dans le support de capsule (3).

9. Inhalateur selon l'une des revendications 1 à 8, pour l'application d'un médicament sous forme pulvérulente ayant un principe actif du groupe des anticholinergiques, des bêta-mimétiques, des stéroïdes, des inhibiteurs de la phosphodiestérase IV, des antagonistes de LTD4, des inhibiteurs de l'EGFR-kinase, des anti-allergiques, des dérivés d'alcaloïdes de l'ergot, de triptans, d'antagonistes de CGRP, d'inhibiteurs de la phosphodiestérase V ainsi que des combinaisons de ces principes actifs.
